# EUROPEAN PATENT APPLICATION

(11) **EP 4 520 827 A1**
(43) Date of publication of application: **12.03.2025**
(21) Application number: 23811131.4
(22) Date of filing: 25.05.2023
(51) Int. Cl.: C12N 15/113, C12Q 1/6883, A61K 31/713, A61K 45/00, A61K 47/46, A61P 9/10

(54) **CIRCULAR RNA CIRC-MAGI1 AND USES THEREOF**

(30) Priority: 26.05.2022 CN 202210582170
(71) Applicant: Orisomes Biotech Co., Ltd, Qingdao, Shandong 266000 (CN)
(72) Inventor: JI, Guangju, Qingdao, Shandong 266000 (CN); LIU, Qun, Qingdao, Shandong 266000 (CN); WANG, Huiwen, Qingdao, Shandong 266000 (CN); YANG, Zhiguang, Qingdao, Shandong 266000 (CN); BI, Youkun, Qingdao, Shandong 266000 (CN)
(74) Representative: Leonard, Thomas Charles
(86) International application number: PCT/CN2023/096287
(87) International publication number: WO 2023/227061

(57) **Abstract**

Provided are circular RNA circ-Magi1 and uses thereof, which belong to the field of circular RNA medical uses. Nucleotide sequences of circular RNA circ-Magi1 are as represented by SEQ ID NO: 1 or SEQ ID NO: 4. Further provided are a pharmaceutical composition for preventing or treating cerebral stroke, uses of a reagent for knockdown, knockout, or silencing of circular RNA circ-Magi1 or a reagent for reducing or inhibiting the expression of circular RNA circ-Magi1 in the preparation of a drug for preventing or treating cerebral stroke, a use of a circular RNA circ-Magi1 gene as a target gene, and a method for preventing or treating cerebral stroke.

## Description

### CROSS REFERENCE TO RELATED APPLICATIONS

This application claims the priority of the Chinese patent application No. 202210582170X, titled "CIRCULAR RNA-CIRC-MAGI1 AND USE THEREOF", filed on May 26, 2022, which is incorporated herein by reference in its entirety.

### TECHNICAL FIELD

The present disclosure relates to the medical field of circular RNA, in particular to a circular RNA-circ-Magi1, a pharmaceutical composition for preventing or treating cerebral stroke, a reagent for knocking down or knocking out or silencing a circular RNA-circ-Magi1, use of a reagent for reducing or inhibiting expression of a circular RNA-circ-Magi1 in preparing a medicament for preventing or treating cerebral stroke, use of a circular RNA-circ-Magi1 gene as a target gene, and a method for preventing or treating cerebral stroke.

### BACKGROUND

Cerebral stroke, also known as stroke, is an acute cerebrovascular disease caused by brain tissue damage due to rupture or blockage of blood vessels in the brain which results in that blood cannot flow to the brain, including ischemic and hemorrhagic strokes. Cerebral stroke has the characteristics of high morbidity, high mortality, high disability rate, high recurrence rate, etc. Cerebral stroke has become the leading cause of death in China as well as the primary cause of disability among Chinese adults.

With the deepening of genomics research, more and more non-coding RNAs have been identified, and their important role in the regulation of gene expression is being recognized. Circular RNA (circRNA), a special class of non-coding RNA, is a non-coding RNA that is covalently closed and looped, which is formed during reverse splicing. In eukaryotes, circular RNAs are expressed abundantly, widely existing in various organs and tissues, exhibit spatiotemporal specificity and tissue specificity, and are also evolutionarily conserved. Circular RNAs, without a 5' cap structure and 3' polyadenylate tail structure, are a closed loop in structure, which is not easily degraded by exonucleases. Therefore, the half-life of most circRNAs exceeds 48 hours, whereas the half-life of mRNA is only 10 hours on average.

It has been reported that circular RNAs are widely involved in the occurrence and development of various diseases, providing theoretical support for the elucidation of the mechanism of the occurrence and development of the diseases, and providing a new research idea for the diagnosis and treatment of various diseases. Circular RNAs have the characteristics of tissue specificity, disease specificity, timing specificity, and high stability, exhibiting an excellent application prospect in terms of diagnostic markers for diseases.

At present, due to the suddenness, urgency, and harmfulness of cerebral stroke, a new treatment method is urgently needed.

### SUMMARY

### OBJECTIVES OF THE INVENTION

An object of the present disclosure is to provide a circular RNA-circ-Magi1, a pharmaceutical composition for preventing or treating cerebral stroke, a reagent for knocking down or knocking out or silencing a circular RNA-circ-Magi1, use of a reagent for reducing or inhibiting expression of a circular RNA-circ-Magi1 in preparing a medicament for preventing or treating cerebral stroke, use of a circular RNA-circ-Magi1 gene as a target gene, and a method for preventing or treating cerebral stroke.

### TECHNICAL SOLUTIONS

To achieve the above objective, according to various embodiments of the present disclosure, the present disclosure provides the following technical solutions.

In a first aspect, the present disclosure provides a circular RNA-circ-Magi1 having a nucleotide sequence as set forth in SEQ ID NO: 1 or SEQ ID NO: 4, wherein SEQ ID NO: 1 is a human circular RNA-circ-Magi1 and specifically has a sequence of:

GCACAACCCGATCTCCCAGAGAAGGAGAAGTGCCTGGCGTGGACTATAACTT
TCTGACTGTGAAGGAGTTCTTGGACCTCGAGCAGAGTGGGACTCTTCTGGAAGTCGGC
ACCTATGAAGGAAACTATTATGGGACACCCAAGCCTCCTAGCCAGCCAGTCAGTGGGAA
AGTGATCACGACGGATGCCTTGCACAGCCTTCAGTCTGGCTCTAAGCAGTCGACCCCGA
AGCGAACCAAGTCCTACAATGATATGCAAAATGCTGGCATAGTCCACGCGGAGAATGAG
GAGGAGGATGACGTTCCTGAAATGAACAGCAGCTTTACAG (SEQ ID NO: 1).

In a second aspect, provided is a pharmaceutical composition for preventing or treating cerebral stroke. The pharmaceutical composition includes a reagent for knocking down or knocking out or silencing or mutating a circular RNA-circ-Magi1, or a reagent for reducing or inhibiting expression of a circular RNA-circ-Magil.

Further, the reagent for knocking down or knocking out or silencing or mutating the circular RNA-circ-Magi1, or the reagent for reducing or inhibiting expression of the circular RNA-circ-Magi1 is at least one selected from the following (a) to (g):
(a) an interfering RNA that knocks down or knocks out the circular RNA-circ-Magi1;
(b) a delivery vector containing any of the interfering RNA in (a);
(c) a miRNA;
(d) an antibody;
(e) an antisense oligonucleotide;
(f) a blocking agent; and
(g) a gene editing system.

Further, the pharmaceutical composition includes an interfering RNA that knocks down or knocks out the circular RNA-circ-Magi1 and a delivery vector containing the interfering RNA. Optionally, the interfering RNA includes siRNA and/or shRNA.

Optionally, the delivery vector is one selected from the group consisting of lipid particles, sugar particles, metal particles, protein particles, liposomes, vesicles, exosomes, a plasmid vector, and a viral vector.

Optionally, a transfection reagent available for clinical injection is also included.

Optionally, the delivery vector is a viral vector, wherein the viral vector is one of a retroviral vector, a lentiviral vector, an adenoviral vector, or an adeno-associated viral vector; and the viral vector is loaded with the interfering RNA.

Optionally, the delivery vector is exosomes, wherein the exosomes are selected from RVG-modified exosomes or nerve cell-specific exosomes, and the exosomes are loaded with the interfering RNA. Optionally, the exosomes are loaded with the interfering RNA by electrical transformation or chemical transformation.

As a feasible embodiment, the siRNA has a nucleotide sequence as set forth in SEQ ID NO: 7, or SEQ ID NO: 13 and/or SEQ ID NO: 14.

Optionally, the shRNA has a sense strand having a nucleotide sequence as set forth in SEQ ID NO: 8, and the shRNA has a sense strand having a nucleotide sequence as set forth in SEQ ID NO: 9.

As a preferred embodiment, the delivery vector is a viral vector. Optionally, the viral vector is loaded with a nucleotide sequence as set forth in SEQ ID NO: 8 and/or SEQ ID NO: 9.

As a preferred embodiment, the delivery vector is exosomes. Optionally, the exosomes are loaded with a nucleotide sequence as set forth in SEQ ID NO: 13 and/or SEQ ID NO: 14.

Optionally, the circular RNA-circ-Magi1 has a nucleotide sequence as set forth in SEQ ID NO: 4.

Further, the siRNA has a nucleotide sequence as set forth in SEQ ID NO: 17 and/or SEQ ID NO: 18, or a nucleotide sequence as set forth in SEQ ID NO: 19 and/or SEQ ID NO: 20.

Further, the circular RNA-circ-Magi1 has a nucleotide sequence as set forth in SEQ ID NO: 1.

Optionally, the cerebral stroke is one or more of ischemic cerebral stroke, hemorrhagic cerebral stroke and lacunar cerebral stroke.

In a third aspect, provided is use of a reagent for knocking down or knocking out or silencing a circular RNA-circ-Magi1, or a reagent for reducing or inhibiting expression of a circular RNA-circ-Magi1 in preparing a medicament for preventing or treating cerebral stroke;
optionally, the circular RNA-circ-Magi1 has a nucleotide sequence as set forth in SEQ ID NO: 1;
and/or the cerebral stroke is one or more of ischemic cerebral stroke, hemorrhagic cerebral stroke and lacunar cerebral stroke.

In a fourth aspect, provided is use of a circular RNA-circ-Magi1 gene as a target gene in following a1) to a4):
a1) preparing a medicament for treatment or auxiliary treatment of cerebral stroke;
a2) screening a medicament for treatment or auxiliary treatment of cerebral stroke;
a3) preparing a medicament for inhibiting or preventing expression of a circular RNA-circ-Magi1; or
a4) preparing a medicament for knocking down, knocking out or silencing the circular RNA-circ-Magil gene.

Further, the circular RNA-circ-Magi1 gene has a nucleotide sequence as set forth in SEQ ID NO: 1.

Optionally, the cerebral stroke is one or more of ischemic cerebral stroke, hemorrhagic cerebral stroke and lacunar cerebral stroke.

In a fifth aspect, provided is a method for preventing or treating cerebral stroke, including administering an effective dose of the pharmaceutical composition for preventing or treating cerebral stroke as described in the fifth aspect to an individual in need thereof.

Optionally, the cerebral stroke is one or more of ischemic cerebral stroke, hemorrhagic cerebral stroke and lacunar cerebral stroke.

One or more embodiments of the present disclosure will be described below in detail. Other features, objects and advantages of the present disclosure will be apparent from the description and claims.

### BRIEF DESCRIPTION OF THE ACCOMPANYING DRAWINGS

In order to better describe and illustrate embodiments and/or illustrations of those inventions disclosed herein, reference may be made to one or more of the accompanying drawings. The additional details or examples used to describe the accompanying drawings should not be considered as limitations to the scope of any of the disclosed inventions, the presently described embodiments and/or examples, and the presently understood best mode of these inventions.
FIG. 1 shows the relative expression levels of circular RNA circ-Magi1 in various organs of mice in Example 1 of the present disclosure.
FIG. 2 shows the relative expression levels of circular RNA circ-Magi1 in various parts of mouse brain tissue in Example 1 of the present disclosure.
FIG. 3 shows the expression levels of circular RNA circ-Magi1 in the HT22 cell line during oxygen-glucose deprivation and during reoxygenation and glucose resupply in Example 2 of the present disclosure.
FIG. 4 shows the relative expression levels of circular RNA circ-Magi1 in H9c2 cell line during oxygen-glucose deprivation and during reoxygenation and glucose resupply in Example 2 of the present disclosure.
FIG. 5 shows the neurobehavioral score of MACO model mice at 24 hours post operation in Example 3 of the present disclosure.
FIG. 6 shows the TTC staining result of the brain of MACO model mice at 24 hours post operation in Example 3 of the present disclosure.
FIG. 7 shows the statistical analysis of the TTC staining result of the brain of MACO model mice at 24 hours post operation in Example 3 of the present disclosure.
FIG. 8 shows the expression level of circular RNA circ-Magi1 in brain of MCAO mice at 24 hours post operation in Example 4 of the present disclosure.
FIG. 9 shows the change in expression level of circular RNA circ-Magi1 in blood of MCAO mice at 24 hours post operation in Example 4 of the present disclosure.
FIG. 10 shows the relationship between the expression level of circular RNA circ-Magi1 in blood and the cerebral ischemia area of MCAO mice at 24 hours post operation in Example 4 of the present disclosure.
FIG. 11 shows the expression levels of circ-Magi1 in blood and blood cells of the control group and the MCAO model group in Example 4 of the present disclosure.
FIG. 12 shows the standard curve for determining virus titer in Example 5 of the present disclosure.
FIG. 13 shows the result of nuclear magnetic resonance of brains of mice in Example 6 of the present disclosure. AAV-NC represents the control virus, and AVV-shRNA represents the experimental virus.
FIG. 14 shows the TTC staining result of brain slices of mice in Example 6 of the present disclosure. AAV-NC represents the control virus, and AVV-shRNA represents the experimental virus.
FIG. 15 shows the result of behavior test of cerebral stroke mice post MCAO operation in Example 6 of the present disclosure. AAV-NC represents the control virus, and AVV-shRNA represents the experimental virus.
FIG. 16 shows the result of rotarod test of cerebral stroke mice post MCAO operation in Example 6 of the present disclosure. AAV-NC represents the control virus, and AVV-shRNA represents the experimental virus.
FIG. 17 shows the test result of exosomes prepared in Example 7 of the present disclosure. Panel A is an image under electron microscope, and panel B is a diagram of particle size analysis.
FIG. 18 shows fluorescence images for different exosomes in Example 8 of the present disclosure. Blank represents non-fluorescent-labeled RVG-Lamp2b-siRNA, Control represents fluorescent-labeled control siRNA-control exosomes, and RVG-Lamp2b represents RVG-Lamp2b-siRNA exosomes (i.e., fluorescent-labeled RVG-Lamp2b-siRNA).
FIG. 19 is a diagram showing the treatment strategy of injecting exosomes loaded with siRNA-NC or siRNA to MCAO mice on day 1, day 2, and day 3 respectively post operation in Example 8 of the present disclosure.
FIG. 20 is a diagram of cylinder test for evaluating the degree of nerve injury in mice in Example 8 of the present disclosure. NC represents healthy mice, 293T-exo represents control siRNA-control exosomes, 293T-exo-siRNA represents RVG-Lamp2b-siRNA exosomes loaded with targeting RNA circ-Magi1.
FIG. 21 is a diagram of adhesive test for evaluating the degree of nerve injury in mice in Example 8 of the present disclosure. NC represents healthy mice, 293T-exo represents control siRNA-control exosomes, 293T-exo-siRNA represents RVG-Lamp2b-siRNA exosomes loaded with targeting RNA circ-Magi1.
FIG. 22 shows the effect of interfering RNA on the expression of circ-Magi1 in the HeLa cell line in Example 9 of the present disclosure. Negative control represents HeLa cell line not transfected with siRNA, Control siRNA represents HeLa cell line transfected with the control siRNA, siRNA1 represents HeLa cell line transfected with siRNA1, and siRNA2 represents HeLa cell line transfected with siRNA2.

### DETAILED DESCRIPTION

In order to make the purpose, technical solutions, and advantages of the embodiments of the present disclosure clearer, the technical solutions in the embodiments of the present application will be clearly and completely described below. Apparently, the described embodiments are part, but not all, of the embodiments of the present disclosure. Based on the embodiments in this application, all other embodiments obtained by persons of ordinary skill in the art without creative efforts fall within the protection scope of the present disclosure. Unless otherwise clearly defined, in the whole description and claims, the terms "comprise" or its variations such as "contain" or "include", etc. will be understood as including the elements or component parts as stated without excluding other elements or other component parts.

In addition, numerous specific details are given in the following detailed description in order to better illustrate the present application. It will be understood by those skilled in the art that the present application may be practiced without certain of the specific details. In some embodiments, materials, components, methods, means, and so on that are well known to those skilled in the art are not described in detail to highlight the gist of the present disclosure.

Hereinafter, the present disclosure will be described in detail.

Magi1 protein belongs to the membrane-associated guanosine kinase family. It participates in the assembly process of polymeric protein complexes and locates at cell junctions, playing a role as a skeleton protein in the establishment of cell junctions, cell shape maintenance and cell signal transduction. Magi1 gene can form various mRNAs thorough alternative splicing during the transcription process, which in turn encodes various subtypes of Magi1. Circ-Magi1 is a circular RNA molecule formed by reverse splicing of exon3 and exon4 of the Magi1 gene. The molecular characteristics and biological functions of circ-Magi1 have not been reported yet.

In the present disclosure, through the qPCR detection method, it was confirmed that circular RNA circ-Magi1, hereinafter referred to as circ-Magi1, was present in the mouse hippocampal neuron cell line HT22. Oxygen-glucose deprivation induced the expression of circ-Magi1, and reoxygenation and glucose resupply induced the further expression of circ-Magi1, showing that the expression of circ-Magi1 in nerve cells is significantly increased under the condition of oxygen-glucose deprivation. However, the expression of circular RNA circ-Magi1 did not change significantly in the rat cardiomyocyte cell line H9c2 under hypoxic conditions, indicating that the expression of circ-Magi1 induced under oxygen-glucose deprivation is specific to nerve cells, which may suggest that the change of expression of circular RNA circ-Magi1 in blood originates from nerve cells. In the established mouse MCAO animal model, the expression level of circ-Magi1 in blood is positively correlated with the cerebral ischemia area, indicating that the expression level of circ-Magi1 is positively correlated with cerebral stroke in MCAO mice, and thus circular RNA circ-Magi1 can be used as a biomarker for cerebral stroke.

Preventive and therapeutic effects on cerebral stroke by knocking down or knocking out circ-Magi1 are shown in the present disclosure.

The present disclosure involves the following nucleotide sequences.
Human circ-Magi1 sequence (SEQ ID NO: 1): 
GCACAACCCGATCTCCCAGAGAAGGAGAAGTGCCTGGCGTGGACTATAACTT
TCTGACTGTGAAGGAGTTCTTGGACCTCGAGCAGAGTGGGACTCTTCTGGAAGTCGGC
ACCTATGAAGGAAACTATTATGGGACACCCAAGCCTCCTAGCCAGCCAGTCAGTGGGAA
AGTGATCACGACGGATGCCTTGCACAGCCTTCAGTCTGGCTCTAAGCAGTCGACCCCGA
AGCGAACCAAGTCCTACAATGATATGCAAAATGCTGGCATAGTCCACGCGGAGAATGAG
GAGGAGGATGACGTTCCTGAAATGAACAGCAGCTTTACAG
Human circ-Magi1 qPCR primers:

| | |
|---|---|
| h-circ-Magi1 (F) (SEQ ID NO: 2) | TCTGCTCGAGGTCCAAGAAC |
| h-circ-Magi1 (R) (SEQ ID NO: 3) | CAGCAGCTTTACAGGCACAACC |

Mouse circ-Magi1 sequence (SEQ ID NO: 4): 
GCACAACCCGGTCTCCCAGAGAAGGAGAAGTGCCTGGTGTGGATTACAGCTT
TCTGACTGTGAAGGAGTTCTTGGACCTCGAGCAGAGCGGGACCCTGTTGGAAGTCGGC
ACCTATGAAGGAAACTATTATGGGACACCCAAACCTCCTAGCCAGCCAGTCAGTGGGAA
AGTGATCACGACGGATGCCTTGCACAGCCTGCAGTCTGGCTCCAAGCAGTCGACCCCTA
AGCGAACAAAGTCCTACAATGATATGCAAAATGCTGGCATAGTCCACCCGGAGAATGAG
GAGGAGGAGGATGTCCCTGAAATGAACAGTAGCTTTACAG
Mouse circ-Magi1 qPCR primers:

| | |
|---|---|
| m-circ-Magi1 (F) (SEQ ID NO: 5) | TCTGCTCGAGGTCCAAGAAC |
| m-circ-Magi1 (R) (SEQ ID NO: 6) | CAGTAGCTTTACAGGCACAACC |

The human circ-Magi1 qPCR primers and the mouse circ-Magi1 qPCR primers were obtained through artificial synthesis.

The cell lines cultivated in the present disclosure are those commercially available. For example, mouse hippocampal neuron cell line HT22 and rat cardiomyocyte cell line H9c2 are commercially available products. Those cell lines from ATCC cell bank can be used.

All of the biological materials, such as mice, used in the present disclosure are commercially available. For example, C57BL male black mice were purchased from Beijing Vital River Laboratory Animal Technology Co., Ltd.

### Example 1 High expression of circ-Magi1 in mouse brain tissue

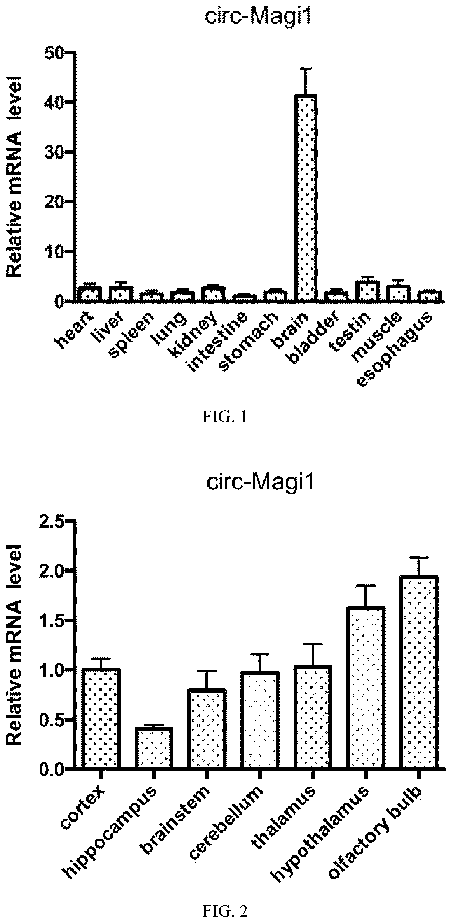

Five 8-week-old C57BL male black mice were selected and sacrificed by cervical dislocation. The heart, liver, spleen, lung, kidney, small intestine, stomach, brain, bladder, testis, skeletal muscle, esophagus and other tissues and organs were collected. RNA was extracted and the expression of circ-Magi1 was measured with mouse circ-Magi1 qPCR primers by using the qPCR method. The result was shown in FIG.1, showing that circ-Magi1 was highly expressed in the brain tissue (i.e., a mixture of various parts of the brain).

For further analyzing the expression of circ-Magi1 in various parts of the brain, another five 8-week-old C57BL male black mice were sacrificed by cervical dislocation and then the following brain structures were collected: cerebral cortex, hippocampus, brainstem, cerebellum, thalamus, hypothalamus, and olfactory bulb. RNA was extracted, and the expression of circ-Magi1 was detected with mouse circ-Magi1 qPCR primers by using the qPCR method. The result was shown in FIG.2, showing a higher expression level in the hypothalamus and olfactory bulb, and a lower expression level in the hippocampus.

### Example 2 Expression of circ-Magi1 in mouse nerve cells during oxygen-glucose deprivation and recovery

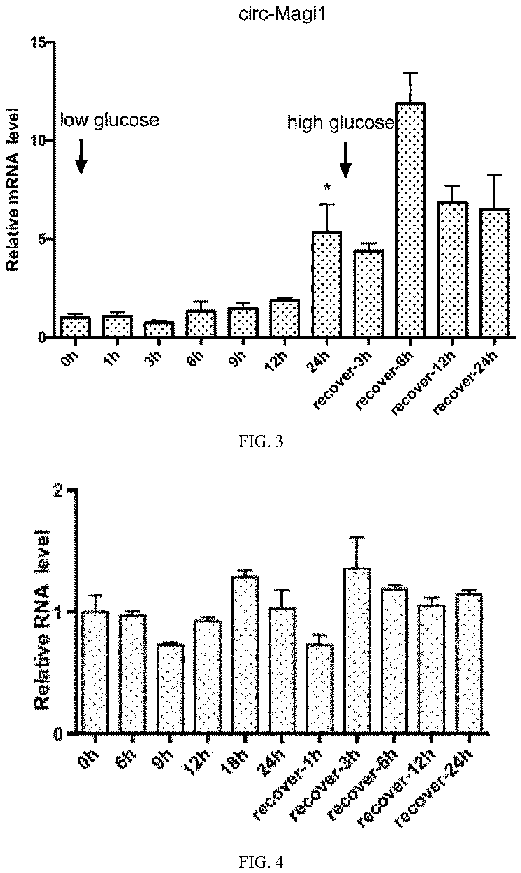

After the cells of mouse hippocampal neuron cell line HT22 were attached to the wall, the culture medium was replaced with a low-glucose medium, and the cells were cultured in a cell incubator with 1% O₂ and 5%CO₂ in saturated humidity for 24 hours. Then, the culture medium was replaced with a high-glucose medium, and the cells were cultured in the cell incubator with 5% CO₂ in saturated humidity for another 24 hours. The expression level of circ-Magi1 was measured at 1 hour, 3 hours, 6 hours, 9 hours, 12 hours, 24 hours after oxygen-glucose deprivation and at 3 hours, 6 hours, 12 hours, 24 hours after reoxygenation. As shown in FIG. 3, the oxygen-glucose deprivation induced the expression of circ-Magi1, and the recovery of oxygen and glucose induced the further expression of circ-Magi1 (n=3). That is, the expression of circ-Magi1 RNA in nerve cells was significantly increased under the condition of oxygen-glucose deprivation. The expression of circ-Magi1 RNA began to double at 12 hours, and reached 5 times at 24 hours, that is, the expression level could reach 2 to 5 times at 12 hours to 24 hours. The expression of circ-Magi1 RNA was further induced to increase after reoxygenation and glucose resupply.

The expression of circ-Magi1 RNA in rat cardiomyocyte cell line H9c2 under the condition of oxygen-glucose deprivation was further studied to verify the expression of circ-Magi1 in the cardiomyocyte cell line. The result was shown in FIG. 4, showing that the expression level of circ -Magi1 in the cardiomyocyte cell line H9c2 was not changed significantly under the hypoxic condition, indicating that hypoxia would not change the expression level of circ-Magi1 in cardiomyocytes.

The result demonstrated that the expression of circ-Magi1 induced by oxygen-glucose deprivation was specific to nerve cells, suggesting that the change of expression level of circ-Magi1 in blood originates from nerve cells.

### Example 3 Establishment of mouse middle cerebral artery occlusion (MCAO) animal model

8-week-old C57BL male black mice were used, with three mice as a control. Eight mice were subjected to the following MCAO surgery for mouse. A 2 cm incision was made in the middle of the neck of mouse, and the right common carotid artery (CCA) was separated and ligated. Then upward, the external carotid artery (ECA) and the internal carotid artery (ICA) were separated. The ECA was ligated at proximal and distal ends, and the ICA was clamped with a vascular clip. A small incision was made between two lines that ligate the ECA to allow a filament to be inserted. The ECA was snipped and transposed. The filament was further inserted into the ICA about 9 mm deep. After 1 hour, the filament was withdrawn. The neck incision of the mouse was sutured.

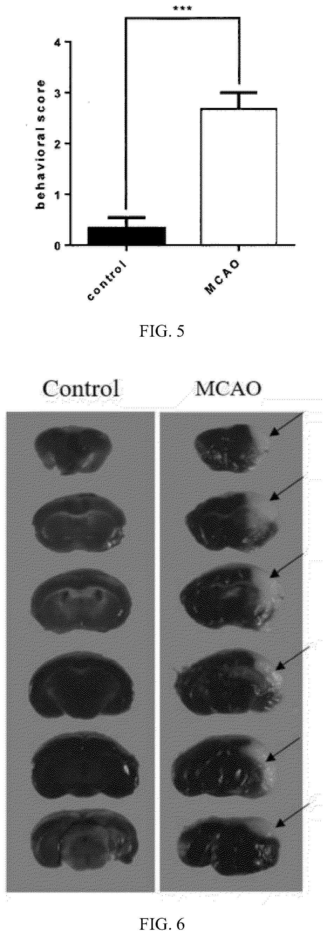

The above-mentioned MCAO mice were behaviorally scored at 24 hours post operation, and the result was shown in FIG. 5. The MACO animal model mice had obvious behavioral disorder, indicating that the MCAO animal model was successfully established.

The degree of cerebral infarction in MCAO model mice was determined by triphenyl tetrazolium chloride (TTC) staining.

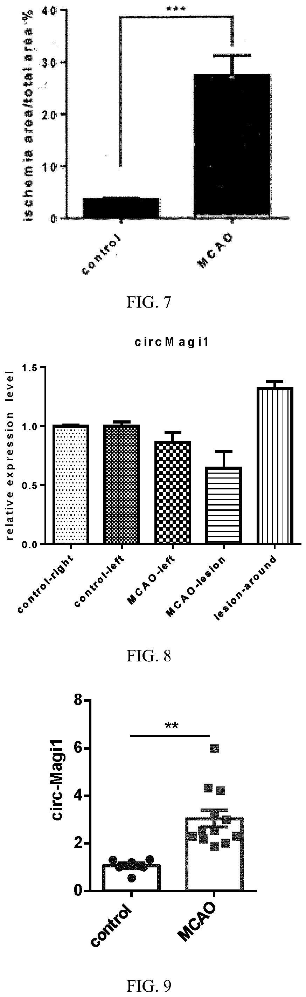

The process of TTC staining was as follows. The animals were sacrificed by brain decapitation. The brains were quickly removed within 10 minutes, and placed in phosphate buffer saline (PBS) solution at 0°C to 4°C for transfer, and frozen in a refrigerator at -20°C for 30 minutes. The cerebellum, olfactory bulb, and lower brainstem were removed. The brains were sliced into coronal brain slices with a thickness of 2 mm, which were placed in 2% triphenyl tetrazolium chloride solution in water bath in dark at 37°C for 30 minutes. The containers were slightly shaken every 5 minutes to ensure fully staining. The brain slices were taken out and washed with PBS solution for 3 to 5 minutes. The brain slices can be photographed immediately, or fixed with 10% neutral formaldehyde for 6 hours. The caudal side of each slice was selected for photographic analysis, and the result was shown in FIG. 6. The infarct area and total area of each slice were measured. The infarct volume of a slice is the product of the infarct area and the thickness of the slice. The sum of the infarct volumes of individual slices is the total infarct volume. The result was shown in FIG. 7.

The result showed that the infarct volume for the MCAO model accounted for about 28% of the total area, significantly higher than that for the control group. The model was successfully established.

### Example 4 Analysis of expression of circ-Magil in brain of MCAO animal model mice

The healthy mice and MCAO animal model mice were analyzed for the expression of circ-Magi1 in brain of the left and right brains of healthy mice, the left brain of MCAO mice, the lesion region of MCAO mice, and the lesion-around region of MCAO mice at 24 hours post operation by the qPCR method. The result was shown in FIG. 8, showing decreased expression of circ-Magi1 in the cerebral stroke lesion region, and increased expression in the lesion-around region.

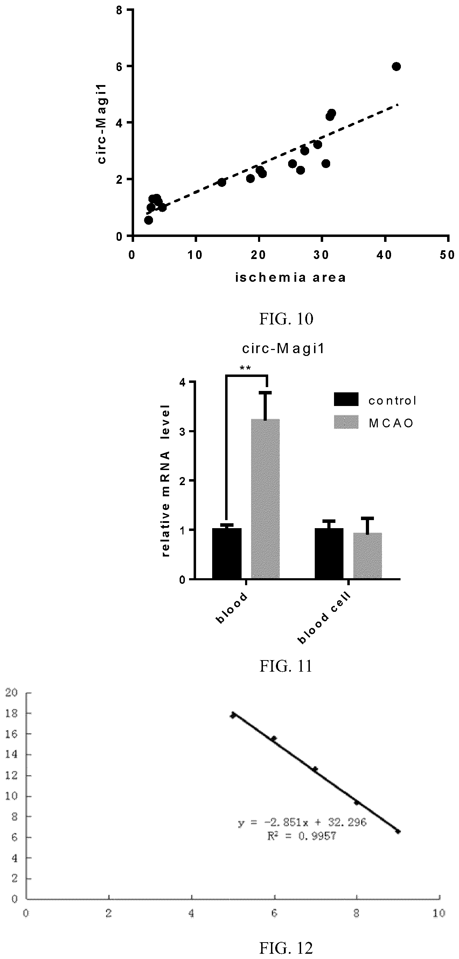

Circular RNA has a biological characteristic of high stability. The expression of RNA circ-Magi1 in the blood of healthy mice and MCAO animal model mice was detected at 24 hours post-operation. The result was shown in FIG. 9, showing that the expression of circ-Magi1 in the blood of MCAO animal model mice was significantly increased. The relationship between the infarct area of mice and the expression level of RNA circ-Magi1 was analyzed. The result was shown in FIG. 10, showing that the expression level of RNA circ-Magi1 in blood was positively correlated with the degree of cerebral stroke in MCAO mice, with R² =0.8824 as shown in FIG. 10.

The expression of circ-Magi1 in blood plasma and blood cells of healthy mice and MCAO animal model mice was further analyzed. The result was shown in FIG. 11, showing that the expression level of circ-Magi1 in the blood cells of MCAO animal model mice was the same as that of healthy mice, and the expression level of RNA circ-Magi1 in the blood of MCAO animal model mice was significantly higher than that of healthy mice, indicating that the change in expression of circ-Magi1 in blood originates from cell-free circ-Magi1 in plasma.

### Example 5 Preparation of shRNA vector

An interference vector pHBAAV-U6-MCS-CMV-EGFP purchased from ATCC was selected. The control viral vector, and the experimental viral vector targeting knockdown or knockout of circular RNA circ-Magi1 were prepared respectively.
1) The siRNA-NC sequence and shRNA-NC sequence in the control viral vectors were as follows.
   siRNA-NC sequence (SEQ ID NO: 10): TTCTCCGAACGTGTCACGTAA
   shRNA-NC sequence
   shRNA-NC sense strand (SEQ ID NO: 11): 
GATCCGTTCTCCGAACGTGTCACGTAATTCAAGAGATTACGTGACACGTTCGG
AGAATTTTTC
   shRNA-NC antisense strand (SEQ ID NO: 12):

AATTGAAAAAATTCTCCGAACGTGTCACGTAATCTCTTGAATTACGTGACACG
TTCGGAGAACG

The shRNA-NC sense and antisense strands (SEQ ID NOs: 11 and 12) were synthesized by Sangon Biotech Co., Ltd. The two shRNA-NC sense and antisense strands were mixed in water with buffer to perform annealing (the reaction system was formulated according to the instruction for buffer), forming a double-stranded fragment with cohesive ends containing enzyme cleavage sites.

2) The siRNA sequence and shRNA1 sequence targeting knockdown or knockout of circular RNA circ -Magi1 in the experimental viral vectors.
siRNA sequence (SEQ ID NO: 7): GCTTTACAGGCACAACCCG
shRNA1 sequence
shRNA1 sense strand (SEQ ID NO: 8): 
AATTCGCTTTACAGGCACAACCCGTTCAAGAGACGGGTTGTGCCTGTAAAGC
TTTTTTG
shRNA1 sense strand (SEQ ID NO: 9):

GATCCAAAAAAGCTTTACAGGCACAACCCGTCTCTTGAACGGGTTGTGCCTG
TAAAGCG

The shRNA1 sense and antisense strands (SEQ ID NOs: 8 and 9) were synthesized by Sangon Biotech Co., Ltd. The two shRNA1 sense and antisense strands were mixed in water with buffer to perform annealing (the reaction system was formulated according to the instruction for buffer), forming a double-stranded fragment with cohesive ends containing enzyme cleavage sites.

3) The double-stranded fragments (i.e., interference fragments) in the above 1) and 2) were each connected to the pHBAAV-T6-MCS-CMV-EGFP vector via enzyme digestion method. Then the vectors were each transformed into DH5α competent cells, and positive clones were screened for sequencing verification. The sequencing result of shRNA1 targeting knockdown or knockout of circular RNA circ-Magi1 was aligned and analyzed as follows.

-----------------aattcgctttacaggcacaacccgttcaagagacgggttgtgcctgtaaagctttttg--------------------------
gaaacaccggtccgcagaattcgctttacaggcacaacccgttcaagagacgggttgtgcctgtaaagctttttggatccattaggcggccgcgtggataa[?]

The sequencing result showed that the sequenced sequence was consistent with the target sequence, suggesting the successful construction of the target plasmid.

### Plasmid extraction

Once the sequencing result showed successful construction, bacterial liquid amplification was performed according to the project requirements, and the plasmids were extracted and purified. The plasmid extraction protocol is subject to the instruction of the extraction kit. The extracted plasmid was used to transfect cells after being qualified by quality control.

### Example 6 Preparation of adeno-associated virus loaded with shRNA

### Cell lines and strains

Packaging cell line: AAV-293 cell purchased from ATCC as a packaging cell of adeno-associated virus, is an attachment-dependent epithelioid cell. The growth medium is Dulbecco's modified eagle medium (DMEM) containing 10% fetal bovine serum (FBS). Adherent cells grew and proliferated in culture to form a monolayer of cells.

Strains: Escherichia coli strain Stbl3 purchased from LabLead, was used to amplify the adeno-associated virus vector and assist to package the vector plasmid.

**Adeno-associated virus packaging system** (i.e., a three-plasmid system): pAAV-RC (purchased from Addgene), pHelper (purchased from Addgene), and a shuttle plasmid which is the target plasmid carrying the shRNA-NC fragment or the shRNA1 fragment prepared in Example 6.

### Adeno-associated virus packaging

Cell Culture: AAV-293 cells were passaged in 100 mm flat dishes for transfection. After the passage, the flat dishes were placed in an incubator with 5% CO₂ at 37°C in 95% relative humidity.

Transfection: The cells can be transfected upon the culture density reached about 80% to 90% confluence. Lipofectamine transfection: Opti MEM was preheated in water bath at 37°C, and Lipofiter^{™} transfection reagent (purchased from Hanbio) was warmed to room temperature and shaken well before use. The composition of the transfection complex required for transfection in a 100 mm flat dish was as follows.

| Composition | Content |
|---|---|
| pAAV-RC plasmid | 10 µg |
| pHelper plasmid | 20 µg |
| shuttle plasmid | 10 µg |
| Lipofiter TM | 120 µg |

Medium replacement: 6 hours after transfection, the medium was replaced with a fresh complete medium containing 10% fetal bovine serum (FBS).

Cell collection: 72 hours after transfection, the cells containing AAV particles were gently scraped off with a cell scraper, collected in a 15 mL centrifuge tube, and centrifuged at 150×g for 3 minutes to collect the cells. After the culture supernatant was discarded, the cells were washed once with PBS solution, and finally resuspended with 300 µL PBS solution.

Cell disruption: Thermostat water bath at 37°C and liquid nitrogen were ready for use. The centrifuge tube containing the cells was subjected to three cycles of freezing and thawing in liquid nitrogen and the water bath at 37°C. The cell lysate was centrifuged at 4°C and 2000×g for 5 minutes to remove cell debris. The lysate supernatant containing AAV particles was collected. Thus, the control virus (denoted as AVV-NC, which was loaded with control shRNA-NC, of which sense and antisense strands were shown in SEQ ID NOs: 8 and 9), and the experimental virus targeting knockdown or knockout of circular RNA circ-Magi1 (denoted as AVV-shRNA, which was loaded with shRNA1 targeting knockdown or knockout of circular RNA circ-Magi, of which sense and antisense strands were as set forth in SEQ ID NOs: 8 and 9).

### Purification of adeno-associated virus

UltraNuclease treatment: 0.1 µL of Benonase enzyme was added to each 1 mL of crude virus extract, followed by incubation in a water bath at 37°C for 1 hour to remove the cellular genome and residual plasmid DNA in the virus extract. The virus extract was centrifuged at 600×g for 10 minutes at 4°C, and the supernatant was collected.

Column purification was performed with Biomiga Adeno-Associated Virus Purification Kit V1469-01.

4 mL of AAV virus sample solution obtained by the column purification was added into an ultrafiltration tube, and centrifuged at 1400×g for 30 minutes, obtaining about 1 mL of AAV. The final purified AAV viruses were collected and stored at -80°C.

### Quality test of adeno-associated virus

The key point of quality control of adeno-associated virus includes sterility test, mycoplasma test and virus titer test.

### Sterility test

Test method: 10 µL of viruses were added to a 96- well plate containing Hela cells for verification. Cells were cultured for 24 hours before inspection under the microscope. Quality control (QC) standard: the medium has to be clear and transparent, with no obvious particles in the intercellular space and no bacterial and fungal contamination.

### Mycoplasma test

Test method: 10 µL of viruses were incubated in a water bath at 96°C for 15 minutes, and then used to prepare a PCR reaction system in a clean bench. After the PCR reaction, electrophoresis was performed to determine whether mycoplasma contamination exists.

QC standard: No obvious band of mycoplasma was observed in the PCR gel electrophoresis map.

### Titer test

The content of AAV genome was measured by fluorescent quantitative Real-time PCR according to SYBRGreen method to determine the AAV titer. The primer sequences of WPRE or ITR, as set forth in SEQ ID NOs: 21 to 24, used in the fluorescent quantitative PCR were as follows.

| Primer | Sequence |
|---|---|
| Forward primer-WPRE | 5'-ACGCTATGTG GATACGCTGC-3' |
| Reverse primer-WPRE | 5'-CGGGCCACAACTCCTCATAA-3' |
| Forward primer-ITR | 5'-CGGCCTCAGTGAGCGA-3' |
| Reverse primer-ITR | 5'-AGGAACCCCTAGTGATG-3' |

### Experimental procedure

1) The AAV virus sample was digested using DNase I and proteinase K.
2) The standard plasmid was diluted to set the copy gradient of 10⁵, 10⁶, 10⁷, 10⁸, 10⁹, and 10¹⁰ to prepare qPCR reaction systems. Three replicate wells were set for each sample and standard. The reaction system was prepared according to the QPCR kit to perform the quantitative PCR reaction. A standard curve was plotted, with the ordinate Y representing the average Ct value of each AAV standard group, and the abscissa X representing the logarithm of the corresponding copy number. As shown in FIG. 12, the formula is y=-2.851x+32.296, R² =0.9957.
3) The titer of the sample to be tested was calculated. The average Ct value of the AAV sample to be tested was substituted into the standard curve formula to calculate the copy number X of the added AAV template. The copy number X was then converted into a titer. The conversion formula was as follows. AAV virus titer = 10^{x} × 40,000 (a dilution factor) vg/mL.

The result was as follows.

The titer of virus AVV-shRNA in the experimental group was 1.2×10¹² vg/mL.

The titer of control virus AAV-NC is 1.6×10¹² vg/mL.

### Example 7 Effect of AAV loaded with shRNA for knockdown or knockout of circ-Magi1 on prevention or treatment of cerebral stroke

Administration dosage: 5 µL of AAV virus with a titer of 1.3×10¹² µg/mL was injected into the lateral ventricle of healthy mice.

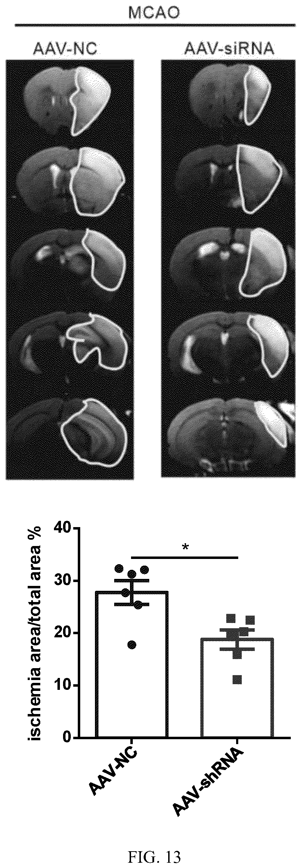

Healthy mice were intracerebroventricularly injected with the virus vector AAV-NC (i.e., a control group) or the virus vector AAV-shRNA (i.e., a treatment group) prepared in Example 7 for 3 weeks before performing MCAO surgery. 3 days later, the mice were subjected to nuclear magnetic resonance. The result was shown in FIG. 13, showing the significantly reduced lesion area of the MCAO mice with knockdown/knockout of circ-Magi1, indicating that knockdown/knockout of circ-Magi1 has a preventive or therapeutic effect on cerebral stroke, and that AAV-shRNA when knocking down/knocking out circ-Magi1, has a preventive or therapeutic effect on cerebral stroke.

Afterwards, mice were dissected to prepare slices for TTC staining.

Staining procedure. Removing the brain was performed by that: after the mouse was anesthetized with 150 µL of 5% chloral hydrate, the brain was directly removed, with the integrity of brain maintained. The brain was quick-frozen in a refrigerator at -20°C for about 20 minutes so as to be sliced easily. Slicing was performed by that: the brain was sliced at intervals of 2 mm. The first cut was done at the midpoint of the line between the anterior pole and the optic chiasm, the second cut was done at the optic chiasm, the third cut was done at the infundibulum handle, and the fourth cut was done between the funnel handle and the caudal pole of the posterior lobe. The slices were placed in TTC solution at a concentration of 2%. After covered with tinfoil, the brain slices were put in an incubator at 37°C for 15 to 30 minutes and frequently turned over to be evenly exposed to the staining solution. The brain slices can also be placed in a multi-well plate, followed by adding dropwise TTC solution, and then the plate was covered with a round cover glass for uniform staining. After that, the slices were taken out, observed, and photographed.

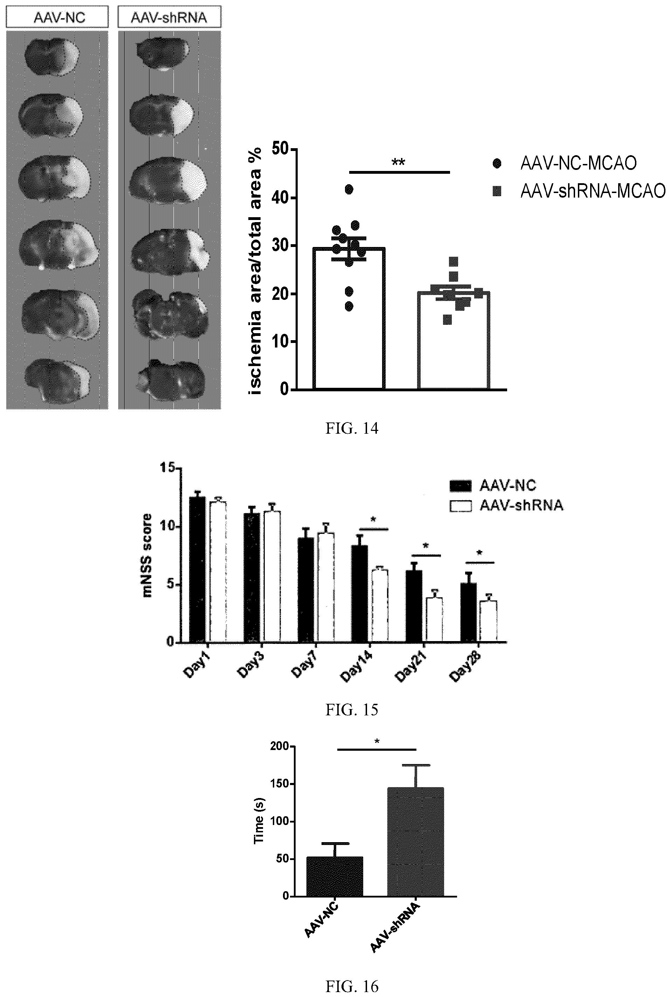

The result was shown in FIG. 14, which is consistent with the NMR result. That is, knocking down circ-Magi1 has a preventive or therapeutic effect on cerebral stroke.

**Behavioral experiment.** The above-mentioned mice were subjected to a behavioral test according to scoring of the modified neurological severity score (mNSS) system.

The mNSS system was designed such that a lower score indicates better function, with a score of 0 indicating full function. The test indicators and scoring were as follows.

The scoring criteria of the mNSS system were as follows.

| | | | |
|---|---|---|---|
| motion test | | | |
| | raising test | | 3 |
| | | flexion of forelimb | 1 |
| | | flexion of hindlimb | 1 |
| | | head movement by >10° with respect to the vertical axis within 30 seconds | 1 |
| | walking test on floor (normal=0; maximum=3) | | 3 |
| | | normal walking | 0 |
| | | inability to walk straight | 1 |
| | | circling toward the paretic side | 2 |
| | | falling on the paretic side | 3 |
| | beam balance test (normal=0; maximum=6) | | 6 |

The behavioral result was shown in FIG. 15. The behavioral scores of both the control group and the treatment group decreased from day 1 to day 28, showing a certain recovery ability after cerebral stroke. The score of the treatment group was significantly lower than that of the control group after 14 days, with a decrease of about 25%, indicating significantly enhanced behavioral recovery ability of the treatment group.

Rotarod test is a test to measure the dwell time of animals on a rotating circular wheel using a mouse rotarod instrument (Model: XR-6C). It is often used in studies of fatigue, brain injury, motor coordination, central nervous system depression, and skeletal muscle relaxation. The mouse was placed on a rotarod with a diameter of 3 cm at a rotation speed of 20 r/min. The time period elapsed from the time when the mouse started to rotate on the rotarod to the time when the mouse dropped from the rotarod was recorded. The mice were trained for 2 days for adapting and learning, and the test was conducted on day 3.

The experimental result was shown in FIG. 16, showing that the mice in the treatment group achieved a dwell time on the rotarod about 150 seconds on day 3, which is significantly higher than the dwell time of about 50 seconds achieved by the mice in the control group, suggesting an enhanced motion ability of the treated mice.

### Example 8 Effect of exosomes loaded with shRNA for knockdown/knockout of circ-Magi1 on prevention or treatment of cerebral stroke

siRNA sequence
Sequences of siRNA targeting circMagi1 loaded in exosomes
Sense strand of siRNA targeting circMagi1 (SEQ ID NO: 13): GCTTTACAGGCACAACCCGTT
Antisense strand of siRNA targeting circMagi1 (SEQ ID NO: 14): CGGGTTGTGCCTGTAAAGCTT
Sequences of control siRNA loaded in exosomes
Sense strand of control siRNA (SEQ ID NO: 15): TTCTCCGAACGTGTCACGTTT
Antisense strand of control siRNA (SEQ ID NO: 16): ACGTGACACGTTCGGAGAATT

The above siRNAs were synthesized by Sangon Biotech Co., Ltd.

293T cell purchased from ATCC is an attachment-dependent epithelioid cell. The growth medium is DMEM containing 10% FBS. Adherent cells grew and proliferated in culture to form a monolayer of cells.

RVG-Lamp-2b plasmid purchased from Addgene carries RVG which is a glycoprotein gene of rabies virus, and has neurotropic specificity.

Strategy: The RVG-Lamp2b plasmid was transfected into 293T cells. After 48 hours, the supernatant was collected to extract exosomes which contain the signal RVG that could reside in the brain. Then, the siRNA capable of knocking down RNA circ-Magi1 was electro-transformed into the exosomes, so that the exosomes could enter the brain and knock down circ-Magi1 in the brain.

### Extraction of exosomes

RVG-Lamp2b exosomes. 48 hours after 293T cells were transfected with the Lamp-2b-RVG plasmid, the supernatant was collected. After centrifugation at 500×g for 5 minutes, the supernatant was collected. After further centrifugation at 2000×g for 10 minutes, the supernatant was collected. After furthermore centrifugation at 10,000×g for 20 minutes, the supernatant was collected. Then, the supernatant was centrifugated at 100,000×g for further 1 hour and 40 minutes, and the pellet was collected and washed with PBS solution once. After centrifugated at 100,000× g for 1 hour and 40 minutes, the pellet was collected and resuspended with an appropriate amount of PBS solution. RVG-Lamp2b exosomes were obtained and stored at -80°C for later use.

Control exosomes. 48 hours after 293T cells were cultured, the supernatant was collected. After centrifugation at 500×g for 5 minutes, the supernatant was collected. After further centrifugation at 2000×g for 10 minutes, the supernatant was collected. After furthermore centrifugation at 10,000×g for 20 minutes, the supernatant was collected. Then, the supernatant was centrifugated at 100,000×g for further 1 hour and 40 minutes, and the pellet was collected and washed with PBS solution once. After centrifugated at 100,000× g for 1 hour and 40 minutes, the pellet was collected and resuspended with an appropriate amount of PBS solution. Control exosomes were obtained and stored at -80°C for later use.

### Identification of exosomes

The following two methods were used to identify RVG-Lamp2b exosomes.
(1) After the exosomes were negatively stained, their morphology was observed with a transmission electron microscope. In the experiment, a copper grid used for the negative staining was subjected to de-static treatment in vacuum for 2 minutes. 5 µl of a diluted sample was added dropwise on the surface of the copper grid, and the excess sample was absorbed with filter paper. 4 µl of uranyl acetate dye solution was added dropwise for staining for 1 minute, and the remaining dye solution was absorbed with filter paper. Then, 4 µl of the dye solution was dropwise added again and discarded after 1 minute. The sample was dried at room temperature, and then observed with the electron microscope.
(2) The exosomes were analyzed for their particle sizes by using dynamic light scattering. With pure water and PBS as controls, 10 µl of a diluted sample was added to a test tube for testing.

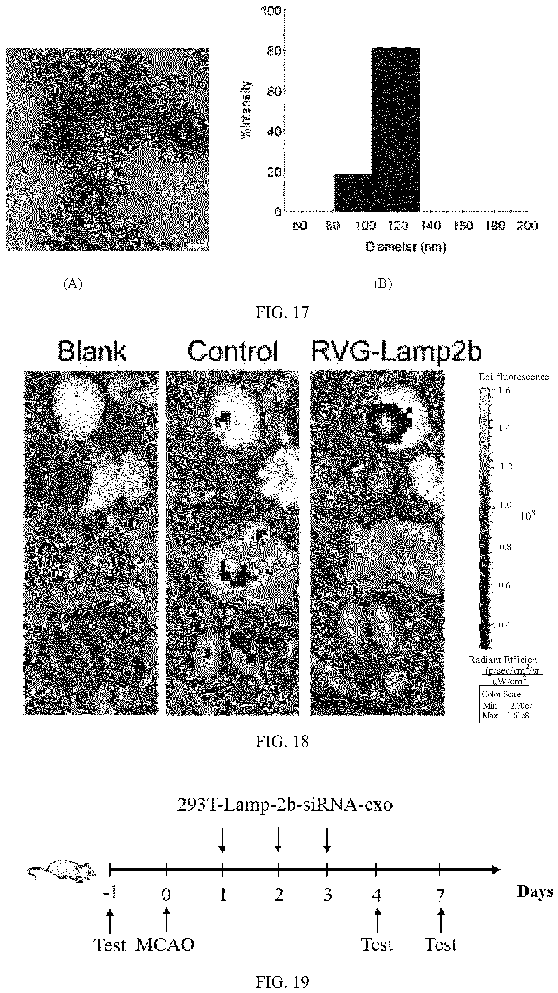

The results were shown in FIG. 17. Panel A, the electron microscope image shows that the RVG-Lamp2b exosomes conform to the morphology of exosomes; and Panel B, the particle size analysis shows that the RVG-Lamp2b exosomes are mainly enriched at 80 nm to 135 nm, which conforms to the diameter of exosomes.

### siRNA electro-transformation for exosomes

After 20 µg of exosomes were mixed with 20 µg of siRNA, the mixture was added to a 1 cm of electroporation cuvette, followed by making up to 400 µl with PBS. The electro-transformation was performed at 400 V and 125 µF, with two pulses at an interval of 5 seconds.

Exosome labeling and biodistribution *in vivo*
(1) After the exosomes were resuspended in 400 µl PBS, 1 ml of Diluent C buffer was added and mixed well.
(2) 4 µl of PKH-67 dye solution was added to 1 ml of Diluent C buffer and mixed well.
(3) The solution in step (1) was added to the solution in step (2), mixed well and incubated at room temperature in the dark for 4 to 10 minutes.
(4) 2 ml of 0.5% BSA/PBS solution was added to stop staining.
(5) The labeled exosomes were ultracentrifuged at 100,000×g at 4°C for 70 minutes by using a SW40 rotor, with the supernatant discarded. The pellet was resuspended in 400 µl of PBS, and then injected into mice via the tail vein.
(6) At 24 hours, 48 hours, and 72 hours after injection of 20 µg of the exosomes via the tail vein, the distribution of exosomes in various organs of the mice was observed using a small animal *in vivo* imager.

The follows were obtained by the above method.

Non-fluorescent-labeled RVG-Lamp2b-siRNA exosomes, i.e., exosomes that were non-fluorescent-labeled RVG-Lamp2b exosomes loaded with siRNA targeting circMagi1. The sense and antisense strands of the siRNA targeting circMagi1 were as set forth in SEQ ID NOs: 13 and 14.

Fluorescent-labeled control siRNA-control exosomes, i.e., exosomes that were fluorescent-labeled control exosomes loaded with control siRNA. The sense and antisense strands of the control siRNA were as set forth in SEQ ID NOs: 15 and 16.

Fluorescent-labeled RVG-Lamp2b-siRNA exosomes, i.e., exosomes that were fluorescent-labeled RVG-Lamp2b exosomes loaded with siRNA targeting circMagi1. The sense and antisense strands of the siRNA targeting circMagi1were as set forth in SEQ ID NOs: 13 and 14.

In this example, non-fluorescent-labeled RVG-Lamp2b-siRNA exosomes, fluorescent-labeled control siRNA exosomes, and fluorescent-labeled RVG-Lamp2b-siRNA exosomes were used for *in vivo* tracing. The results after 24 hours were shown in FIG. 18, showing that the non-labeled RVG-Lamp2b-siRNA exosomes did not have red fluorescence, and that the fluorescent-labeled control siRNA-control exosomes had red fluorescence, which was distributed in brain, liver, kidney, and bladder tissues. The fluorescent-labeled RVG- Lamp2b-siRNA exosomes showed distribution only in the brain. This suggests that the fluorescent-labeled RVG-Lamp2b-siRNA exosomes loaded with the siRNA were optimal for precise drug delivery.

### Treatment experiment

RVG-Lamp2b exosomes loaded with siRNA were used for treatment of cerebral stroke. Administration route includes injection of mice via tail vein once a day for 3 days after the construction of MCAO model, and 20 µg each time.

Treated mice: MCAO model mice were injected with RVG-Lamp2b-siRNA exosomes loaded with siRNA targeting circMagi1 (the sense and antisense strands of which were as set forth in SEQ ID NOs: 13 and 14) on day 1, day 2, and day 3 respectively post operation. A behavioral test was conducted on day -1, day 4, and day 7. The treatment strategy was shown in FIG. 19. Knockdown/knockout of circ-Magi1 has a preventive or therapeutic effect on cerebral stroke.

Control mice: MCAO model mice were injected with RVG-Lamp2b-control siRNA exosomes loaded with control siRNA (the sense and antisense strands of which were as set forth in SEQ ID NOs: 15 and 16) on day 1, day 2, and day 3 respectively post operation. A behavioral test was conducted on day -1, day 4, and day 7.

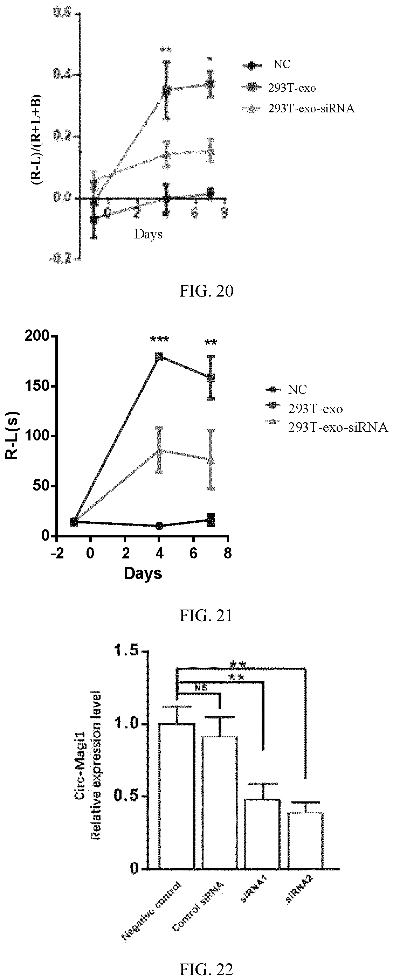

Cylinder test: The mice were placed in a transparent cylinder with a bottom diameter of 15 cm, and observed for the proportion of each side of forelimbs touching the wall within 20 times to evaluate the degree of nerve damage. The results were shown in FIG. 20, showing that there was significance between the number of touches in the affected part of the MCAO control group, which was significantly increased, and the number of touches in the affected part of the treatment group, which was significantly reduced.

Adhesive test: The forelimbs of the mice were stuck with a round adhesive tape, and the time for the mice to break free was evaluated. The result was shown in FIG. 21. Compared with the MCAO model control mice, the treated mice costed significantly reduced time to break free, reflecting better movement coordination and better recovery of cerebral stroke.

### Example 9 Knockdown of Circ-Magi1 in HeLa cells by siRNA targeting human Circ-Magi1

Sense strand of siRNA1 targeting human Circ-Magi1 (SEQ ID NO: 17):
   GACTCTTCTGGAAGTCGGCACCTATTT
Anti-sense strand of siRNA1 targeting human Circ-Magi1 (SEQ ID NO: 18):
   ATAGGTGCCGACTTCCAGAAGAGTCTT
Sense strand of siRNA2 targeting human Circ-Magi1 (SEQ ID NO: 19):
   ACTCTTCTGGAAGTCGGCACCTATGTT
Anti-sense strand of siRNA2 targeting human Circ-Magi1 (SEQ ID NO: 20):
   CATAGGTGCCGACTTCCAGAAGAGTTT
Sense strand of control siRNA (SEQ ID NO: 15): TTCTCCGAACGTGTCACGTTT
Antisense strand of control siRNA (SEQ ID NO: 16): ACGTGACACGTTCGGAGAATT

The above siRNAs were synthesized by Sangon Biotech Co., Ltd, and annealed for later use.

HeLa cell line was from ATCC, cultured in HDMEM with 10% fetal bovine serum.

The HeLa cell line was transfected with siRNA1 (sense and antisense strands as set forth in SEQ ID NOs: 17 and 18, respectively), siRNA2 (sense and antisense strands as set forth in SEQ ID NOs: 19 and 20, respectively), and control siRNA (sense and antisense strands as set forth in SEQ ID NOs: 15 and 16, respectively) respectively by using Lipofetamin2000 reagent (Thermo Fisher), and cultured in HDMEM with 10% fetal bovine serum for 48 hours. Then, RNA was extracted by using the Trizol kit (Thermo Fisher), and the extracted RNA was reverse transcribed by using the reverse transcription kit (Takara). The primers in Example 1 (h-circ-Magi1 (F) (SEQ ID NO: 2), and h-circ-Magi1 (R) (SEQ ID NO: 3)) were used to perform the qPCR detection method. The expression levels of Circ-Magi1 in HeLa cell lines without transfection of siRNA (i.e., a negative control), with transfection of control siRNA, with transfection of siRNA1, and with transfection of siRNA2 were tested respectively, and the results were shown in FIG. 22. The results showed that siRNA1 and siRNA2 successfully reduced the expression of circ-Magi1 in the HeLa cell line by about 50%, which was significant, and that there was no significant change in the control siRNA group compared with the negative control group. This suggests that siRNA1and siRNA2 can knock out/knock down the expression of circ-Magi1, which provides feasibility for the treatment of cerebral stroke.

Finally, it should be noted that the above embodiments are only used to illustrate the technical solutions of the present application, but not limit thereto. Although the present application has been described in detail with reference to the foregoing embodiments, those skilled in the art should understand that the technical solutions described in the foregoing embodiments can still be modified, or some technical features thereof can be equivalently replaced. However, these modifications or substitutions do not make the essence of the corresponding technical solutions deviate from the spirit and scope of the technical solutions of the embodiments of the present application.

## Claims

1. A circular RNA-circ-Magi1 having a nucleotide sequence as set forth in SEQ ID NO: 1 or SEQ ID NO: 4.

2. A pharmaceutical composition for preventing or treating cerebral stroke, comprising a reagent for knocking down or knocking out or silencing or mutating a circular RNA-circ-Magi1, or a reagent for reducing or inhibiting expression of a circular RNA-circ-Magi1.

3. The pharmaceutical composition for preventing or treating cerebral stroke according to claim 2, wherein the reagent is at least one selected from the following (a) to (g):
(a) an interfering RNA that knocks down or knocks out the circular RNA-circ-Magi1;
(b) a delivery vector containing any of the interfering RNA in (a);
(c) a miRNA;
(d) an antibody;
(e) an antisense oligonucleotide;
(f) a blocking agent; and
(g) a gene editing system.

4. The pharmaceutical composition according to claim 2 or 3, wherein the pharmaceutical composition comprises an interfering RNA that knocks down or knocks out the circular RNA-circ-Magi1 and a delivery vector containing the interfering RNA; optionally, the interfering RNA comprises siRNA and/or shRNA;
optionally, the delivery vector is one selected from the group consisting of lipid particles, sugar particles, metal particles, protein particles, liposomes, vesicles, exosomes, a plasmid vector, and a viral vector;
optionally, the pharmaceutical composition further comprises a transfection reagent available for clinical injection;
optionally, the delivery vector is a viral vector, wherein the viral vector is at least one selected from a retroviral vector, a lentiviral vector, an adenoviral vector, or an adeno-associated viral vector; and the viral vector is loaded with the interfering RNA;
optionally, the delivery vector is exosomes, wherein the exosomes are selected from RVG-modified exosomes or nerve cell-specific exosomes, and the exosomes are loaded with the interfering RNA;
optionally, the exosomes are loaded with the interfering RNA by electrical transformation or chemical transformation.

5. The pharmaceutical composition according to claim 4, wherein the siRNA has a nucleotide sequence as set forth in SEQ ID NO: 7, or SEQ ID NO: 13 and/or SEQ ID NO: 14;
optionally, the shRNA has a sense strand having a nucleotide sequence as set forth in SEQ ID NO: 8, and the shRNA has a sense strand having a nucleotide sequence as set forth in SEQ ID NO: 9;
optionally, the delivery vector is a viral vector, wherein the viral vector is loaded with a nucleotide sequence as set forth in SEQ ID NO: 8 and/or SEQ ID NO: 9;
optionally, the delivery vector is exosomes, wherein the exosomes are loaded with a nucleotide sequence as set forth in SEQ ID NO: 13 and/or SEQ ID NO: 14;
optionally, the circular RNA-circ-Magi1 has a nucleotide sequence as set forth in SEQ ID NO: 4.

6. The pharmaceutical composition according to claim 4, wherein the siRNA has a nucleotide sequence as set forth in SEQ ID NO: 17 and/or SEQ ID NO: 18, or a nucleotide sequence as set forth in SEQ ID NO: 19 and/or SEQ ID NO: 20;
and/or the circular RNA-circ-Magi1 has a nucleotide sequence as set forth in SEQ ID NO: 1;
and/or the cerebral stroke is one or more of ischemic cerebral stroke, hemorrhagic cerebral stroke and lacunar cerebral stroke.

7. Use of a reagent for knocking down or knocking out or silencing a circular RNA-circ-Magi1, or a reagent for reducing or inhibiting expression of a circular RNA-circ-Magi1 in preparing a medicament for preventing or treating cerebral stroke;
optionally, the circular RNA-circ-Magi1 has a nucleotide sequence as set forth in SEQ ID NO: 1;
and/or the cerebral stroke is one or more of ischemic cerebral stroke, hemorrhagic cerebral stroke and lacunar cerebral stroke.

8. Use of a circular RNA-circ-Magi1 gene as a target gene in following a1) to a4):
a1) preparing a medicament for treatment or auxiliary treatment of cerebral stroke;
a2) screening a medicament for treatment or auxiliary treatment of cerebral stroke;
a3) preparing a medicament for inhibiting or preventing expression of a circular RNA-circ-Magi1; or
a4) preparing a medicament for knocking down, knocking out or silencing the circular RNA-circ-Magil gene.

9. The use according to claim 8, wherein the circular RNA-circ-Magi1 gene has a nucleotide sequence as set forth in SEQ ID NO: 1;
optionally, the cerebral stroke is one or more of ischemic cerebral stroke, hemorrhagic cerebral stroke, and lacunar cerebral stroke.

10. A method for preventing or treating cerebral stroke, comprising administering an effective dose of the pharmaceutical composition for preventing or treating cerebral stroke according to any one of claims 2 to 6 to an individual in need thereof;
optionally, the cerebral stroke is one or more of ischemic cerebral stroke, hemorrhagic cerebral stroke, and lacunar cerebral stroke.
